# EUROPEAN PATENT SPECIFICATION

(11) **EP 2 565 616 B1**
(45) Date of publication and mention of the grant of the patent: **05.08.2020**
(21) Application number: 11775125.5
(22) Date of filing: 28.04.2011
(51) Int. Cl.: G01N 1/22, G01N 1/28, G01N 27/28, G01N 27/30, G01N 27/416, G01N 33/53, G01N 33/543, G01N 35/10, G01N 37/00, B01L 3/00, G01N 33/00

(54) **CHEMICAL SENSOR**
CHEMISCHER SENSOR
DÉTECTEUR CHIMIQUE

(30) Priority: 28.04.2010 JP 2010104252
(43) Date of publication of application: 06.03.2013
(73) Proprietor: Panasonic Intellectual Property Management Co., Ltd., Osaka-shi, Osaka 540-6207 (JP)
(72) Inventor: TACHIBANA, Hiroaki, Osaka 540-6207 (JP); TSUJI, Koji, Osaka 540-6207 (JP)
(74) Representative: Appelt, Christian W.
(86) International application number: PCT/JP2011/060404
(87) International publication number: WO 2011/136344

(56) References cited:
- WO-A1-2008/021617
- WO-A1-2009/057256
- WO-A2-2006/114335
- WO-A2-2009/020479
- JP-A- 9 243 590
- JP-A- 2001 527 220
- JP-A- 2003 106 961
- JP-A- 2008 051 803
- US-A1- 2007 048 746
- US-A1- 2007 173 731

## Description

### [Technical Field]

The present invention relates to a chemical sensor that detects analytes such as gas components in a gas, viruses, and allergens.

### [Background Art]

There has hitherto been known an electrochemical biosensor test strip in which body fluids such as blood, and fermented or naturally occurring materials are used as a sample and the sample is enzymatically reacted with a reagent, and then charges thus generated are detected (see Patent Literature 1).

It is disclosed that this electrochemical biosensor test strip is formed with a capillary space for the introduction of a sample using a capillary action, and thus enabling an enzymatic reaction with a reagent and precise determination of charges even in the case of a small amount of the sample.

### [Citation List]

### [Patent Literature]

[Patent Literature 1]
JP 2002-541453 W

### [Summary of Invention]

### [Technical Problem]

In the above-mentioned electrochemical biosensor test strip, the sample should be liquid due to restrictions on the reaction. Therefore, it is impossible to detect analytes such as gas components in a gas, viruses, and allergens.

In order to detect analytes such as gas components in a gas, viruses, and allergens, it becomes necessary to carry out a pretreatment in which analytes are dissolved in a liquid. This pretreatment is a complicated treatment that is carried out using a large-sized apparatus, or carried out by manual labor.

WO2006/114335 A2 discloses a method for analyzing components of exhaled air with the aid of electrochemical sensors and/or biosensors. The substances that are to be analyzed are also condensed when the water contained in breathing gas is condensed on a functionalized condensation surface. The obtained condensate is fed directly to the sensors. WO 2009/020479 A2 shows a fluidic device comprising an excitation area, wherein electromagnetic energy excites a SERS probe in a fluid,and a detection area, wherein an emitted spectra is detected by a detection device.

The present invention has been made in view of the above problems and an object thereof is to provide a chemical sensor that can dissolve analytes in a gas in a liquid using a simple method.

### [Solution to Problem]

A chemical sensor according to the feature of the present invention includes a sensor support section; a gas injection hole through which a gas containing an analyte is injected; a sample collection section in which the analyte contained in the gas injected through the gas injection hole is collected; a sample delivery section through which the analyte collected in the sample collection section is delivered to a sample reaction section; the sample reaction section including a reactant that causes a predetermined reaction with the analyte delivered through the sample delivery section; and a detection section in which the analyte reacted with the reactant in the sample reaction section is detected. The sample collection section includes a Peltier element that liquefies water vapor contained in the gas injected through the gas injection hole.

According to the feature of the present invention, it is possible to prepare a solution containing an analyte by liquefying water vapor contained in the gas injected through the gas injection hole due to the Peltier cooling effect of the Peltier element. Therefore, it is possible to collect the solution containing an analyte from the gas injected through the gas injection hole, in the sample collection section.

In the feature of the present invention, preferably, the gas injection hole, the sample collection section, the sample delivery section, the sample reaction section and the detection section are integrally formed on the sensor support section. Thereby, miniaturization and weight saving are possible, and also it becomes possible to detect at low costs.

In the feature of the present invention, the sample delivery section may have a capillary structure, and one end of this capillary structure may be close to the Peltier element. Thereby, it is possible to deliver a solution liquefied by the Peltier element through a capillary phenomenon of the capillary structure to a sample reaction section without using a special structure.

The sample reaction section may include at least any one of an antibody, an enzyme, a receptor and a protein, that specifically reacts with the analyte delivered through the sample delivery section. Thereby, it is possible to specifically detect the analyte by an antigen-antibody reaction with the analyte.

The sample reaction section includes a heating section in which the analyte delivered through the sample delivery section is heated, and a temperature measurement section in which the temperature of the analyte is measured. Since a PCR method for amplifying DNAs of the analyte can be carried out by allowing a specific temperature cycle to occur in the analyte, it is possible to detect the analyte with high sensitivity.

The detection section may include an antibody that binds to the analyte reacted with a reactant, and an electrode that supports the antibody, and this electrode may have a porous structure. Thereby, it is possible to increase an immobilization area of the antibody, and thus enabling an increase in detection sensitivity.

The chemical sensor is composed of a plurality of unit sensors formed integrally on one sensor support section, and each unit sensor may consist of a gas injection hole, a sample collection section, a sample delivery section, a sample reaction section and a detection section. Integral formation of a plurality of unit sensors in one sensor support section enables suppression of single-use of the sensor.

### [Advantageous Effects of Invention]

As described above, according to the chemical sensor of the present invention, it is possible to dissolve analytes in a gas in a liquid due to the Peltier cooling effect of a Peltier element, using a simple method.

### [Brief Description of Drawings]

Fig. 1(a) is a sectional view illustrating the entire configuration of a chemical sensor according to a first embodiment of the present invention, only covered by the present invention when comprising a heating section and a temperature section in the sample reaction section as defined in claim 1.
Fig. 1(b) is a schematic view illustrating a Peltier element with a sample collection section 13 of Fig. 1(a).
Fig. 2(a) and Fig. 2(b) are schematic views, each illustrating a specific configuration example of a sample reaction section 15 and a detection section 16 of Fig. 1(a).
Fig. 3(a) and Fig. 3(b) are schematic views, each illustrating a specific configuration example of the detection section 16 of Fig. 1(a).
Fig. 4(a) and Fig. 4(b) are schematic views, each illustrating a specific configuration example of the sample reaction section 15 according to a second embodiment of the present invention.
Fig. 5 is a conceptual view illustrating the entire configuration of a chemical sensor according to a third embodiment of the present invention, only covered by the present invention when comprising a heating section and a temperature section in the sample reaction section as defined in claim 1.

### [Description of Embodiments]

Embodiments of the present invention will be described below with reference to the accompanying drawings. In the description of drawings, the same reference signs are used for the same structural elements.

### (First Embodiment)

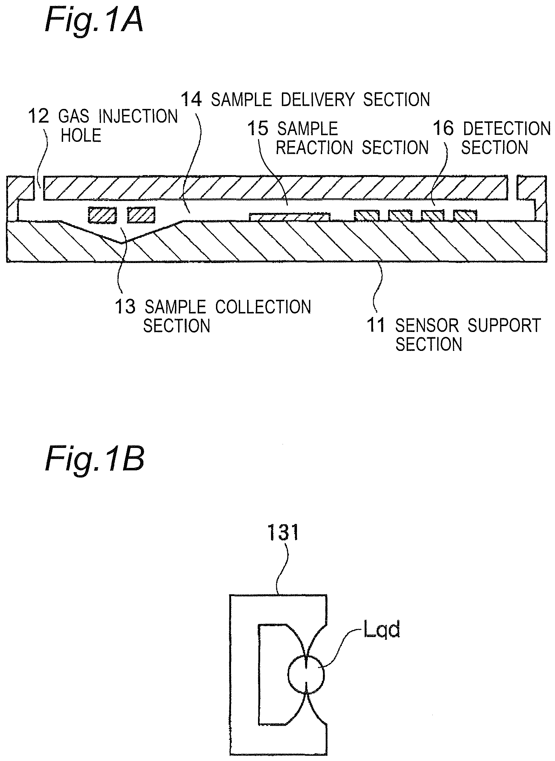

The entire configuration of a chemical sensor according to a first embodiment of the present invention will be described with reference to Fig. 1(a). The chemical sensor according to the first embodiment includes a sensor support section 11 having a function as a substrate that supports the entire chemical sensor; a gas injection hole 12 through which a gas containing an analyte, such as air is injected; a sample collection section 13 in which the analyte contained in the gas injected through the gas injection hole 12 is collected; a sample delivery section 14 through which the analyte collected in the sample collection section 13 is delivered to a sample reaction section 15; the sample reaction section 15 including a reactant that causes a predetermined reaction with the analyte delivered through the sample delivery section 14; and a detection section 16 in which the analyte reacted with the reactant in the sample reaction section 15 is detected.

The sensor support section 11 is a substantially flat substrate and is made, for example, of a semiconductor (Si, SiC, etc.) substrate or a glass substrate that is easy to undergo microprocessing. The sensor support section 11 and the below-mentioned protective member can be produced by micromachining. On one main surface of the sensor support section 11, a sample collection section 13, a sample delivery section 14, a sample reaction section 15 and a detection section 16 are disposed in order. A protective member is disposed over a main surface of the sensor support section 11 so as to cover the sample collection section 13, the sample delivery section 14, the sample reaction section 15 and the detection section 16, and a hole is opened at at least two positions of the protective member. These holes at at least two positions are configured to introduce and discharge a gas containing an analyte, and the protective member and the sensor support section 11 are preferably bonded by a bonding method capable of maintaining airtightness so as to enable effective introduction and discharge of the gas through holes at two positions. One of holes at at least two positions is, for example, provided ahead of the sample collection section 13, and the gas is introduced into the protective member through at least one hole and is discharged through another holes. One hole, through which the gas is introduced, is called a gas injection hole 12. In this way, in a detection space formed by covering the sensor support section 11 with the protective member provided with the gas injection hole 12, a sample collection section 13 in which the analyte contained in the gas is collected in a solution, a sample delivery section 14, a sample reaction section 15 and a detection section 16 are integrally formed.

The sample collection section 13 is disposed close to the gas injection hole 12, thereby facilitating collection of the analyte contained in the gas injected through the gas injection hole 12. Herein, examples of the "analyte contained in a gas" include gas components in a gas, viruses, allergens and the like.

In Fig. 1(a), there has been shown an example in which the gas injection hole 12 is provided ahead upstream of the sample collection section 13. As mentioned above, in the present invention, the sample collection section 13 may be disposed closed to the gas injection hole 12 and, for example, the gas injection hole 12 may be provided on the sample collection section 13.

The sample collection section 13 includes a Peltier element 131 that liquefy water vapor contained in the gas injected through the gas injection hole 12, as shown in Fig. 1(b). By applying a current to the bonded section of two kinds of metals that constitute the Peltier element 131, one metal of the Peltier element 131 absorbs heat from an ambient gas. Fig. 1(b) is a plan view in which the sample collection section 13 shown in Fig. 1(a) is seen from the above, and the lower side of Fig. 1(b) is the gas injection hole 12 side in Fig. 1(a) while the upper side of Fig. 1(b) is the sample delivery section 14 side in Fig. 1(a). In Fig. 1(b), metal of the low temperature side (endothermic side) is shown, and a solution Lqd liquefied from the gas is formed at protrusions provided oppositely and is dropped on a recess provided in the sensor support section 11 directly thereunder. The Peltier element 131 constituting this sample collection section 13 is, for example, supported on the sensor support section 11 at both ends so as not to inhibit a cooling function, and a current is, for example, injected into the bonded section of two kinds of metals through the wiring formed on a support structure thereof.

It is preferred that a Peltier material is, for example, copper oxide, polysilicon thin film or the like from the viewpoint of miniaturization. In this way, according to the present invention, it is possible to liquefy water vapor contained in the gas injected through gas injection hole due to the Peltier cooling effect of the Peltier element. Since the analyte is dissolved in the liquefied water vapor, it is possible to prepare a solution Lqd containing the analyte. Therefore, the sample collection section 13 can automatically collect the solution Lqd containing the analyte from the gas injected through the gas injection hole 12.

The sample delivery section 14 has a capillary structure measuring about 100 µm in width and depth, and one end of the capillary structure is closed to the Peltier element of the sample collection section 13. It is possible to introduce the solution Lqd containing the analyte collected by the Peltier element into the capillary structure. The solution Lqd introduced into the capillary structure is delivered to the sample reaction section 15 by a capillary phenomenon. In this way, the sample delivery section 14 can deliver the solution liquefied by the Peltier element through a capillary phenomenon of the capillary structure to the sample reaction section 15 without using a special structure. The capillary structure measuring about 100 µm in width can be produced by micromachining.

A capillary surface is preferably subjected to a hydropholization treatment so as to facilitate the movement of the solution Lqd. For example, it is easy to produce the capillary structure in the case of a method in which a silicon oxide film is formed on a surface, or a method in which a surface is covered with a water-soluble polymer such as polyethylene glycol.

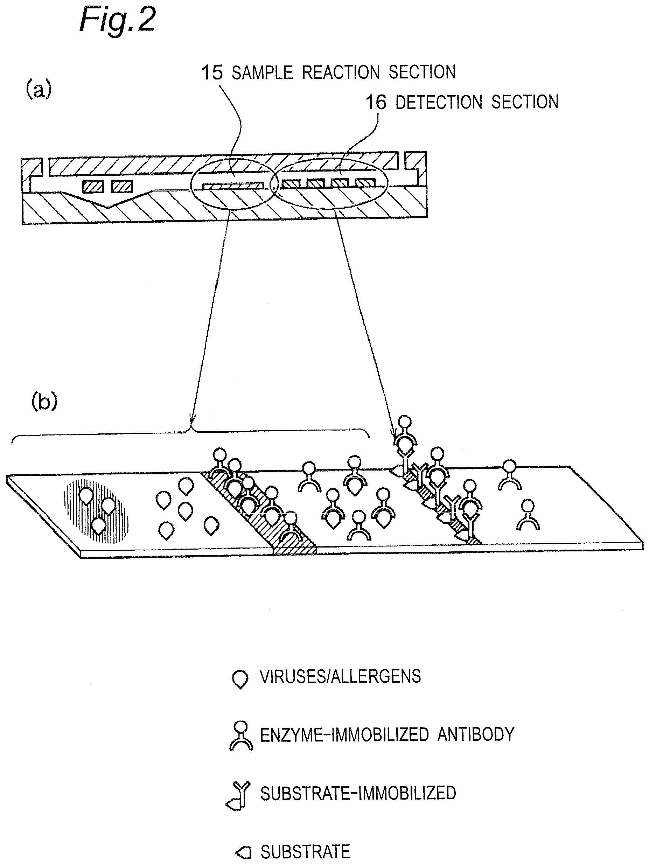

Specific configuration example of a sample reaction section 15 and a detection section 16 of Fig. 1(a) will be described with reference to Fig. 2(a) and Fig. 2(b).

The sample reaction section 15 includes, as a reactant that causes a predetermined reaction with the analyte delivered through the sample delivery section 14, at least any one of an antibody, an enzyme, a receptor and a protein. In the first embodiment, a description will be made on the case of including an enzyme-immobilized antibody as an example of an antibody that specifically reacts with analytes such as viruses and allergens. The enzyme-immobilized antibody specifically binds to viruses or allergens to form a complex. This complex is delivered to the detection section 16. The enzyme-immobilized antibody includes, for example, an anti-HA antibody and an ALP enzyme.

The detection section 16 includes an electrode composed of a metal thin film of gold, platinum or the like, and a substrate-immobilized antibody is supported as an example of an antibody that binds to the above-mentioned complex at a capture site. The substrate-immobilized antibody includes, for example, an anti-HA antibody, and the substrate includes, for example, para-aminophenyl phosphate (pAPP) as a substrate that specifically reacts with an ALP enzyme. The substrate-immobilized antibody can capture viruses or allergens that bind to the enzyme-immobilized antibody. Thereby, the substrate of the substrate-immobilized antibody is close to an enzyme of an enzyme-immobilized antibody at the capture site, where an enzyme-substrate reaction arises. In case the enzyme is an ALP enzyme and the substrate is pAPP, para-aminophenol (pAP) is formed by the reaction. The thus formed pAP can be oxidized to para-quinoneimine (pQI) on the electrode. The detection section 16 can detect the analyte by measuring electrons that move by the oxidation reaction, using the electrode. Since an oxidation-reduction reaction of pAP with pQI is reversible, an oxidation-reduction cycle can be formed by forming the electrode in an opposing comb shape, and oxidizing pAP while reducing pQI, and thus enabling amplification of the obtained current value. Thereby, it becomes possible to detect with high sensitivity.

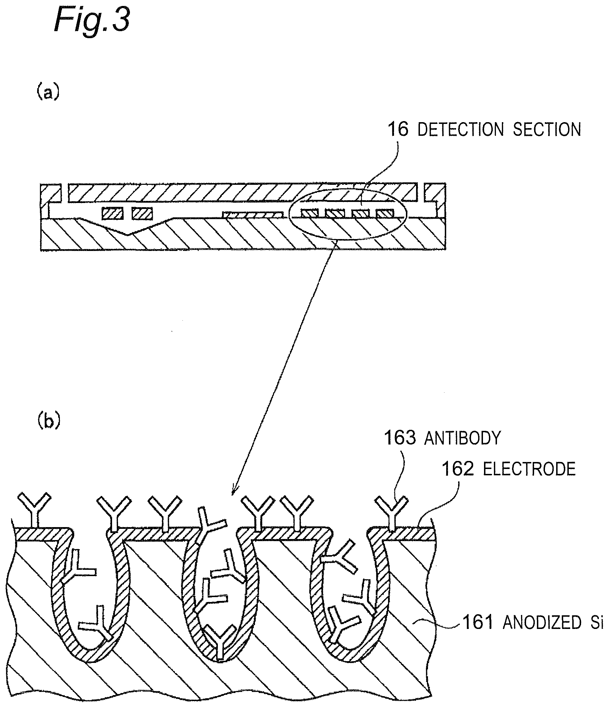

Specific configuration example of the detection section 16 of Fig. 1(a) will be described with reference to Fig. 3(a) and Fig. 3(b). The detection section 16 includes an antibody (substrate-immobilized antibody) 163 that binds to analytes (viruses, allergens, etc.) reacted with a reactant (enzyme-immobilized antibody), an electrode 162 that supports the antibody 163, and an anodized silicon 161.

On a main surface of the anodized silicon 161, a plurality of recesses are formed. The electrode 162 is composed of a thin film of gold (Au) formed in a uniform thickness along an irregular shape of the main surface of this anodized silicon 161. In such way, the electrode 162 has a porous structure. On a surface of the electrode 162, an antibody 163 is supported. Since the antibody 163 is also supported in a recess, the detection section 16 can also support a lot of antibodies 163. When the electrode 162 has a porous structure, it is possible to increase an immobilization area of the antibody 163, and thus enabling an increase in detection sensitivity. It may be possible to use, as an immobilization method of the antibody 163, for example, a method of a self-assembled monolayer (SAM) that binds to a metal surface via a thiol group.

As described above, according to the first embodiment of the present invention, the following operation and effect can be obtained.

As illustrated in Fig. 1, a solution containing analytes (viruses, allergens, etc.) can be prepared by liquefying water vapor contained in a gas injected through a gas injection hole 12 due to the Peltier cooling effect of the Peltier element. Therefore, the sample collection section 13 can collect the analyste from the gas injected through the gas injection hole 12.

In the chemical sensor according to the present invention, a sample delivery section 14, a sample reaction section 15 and a detection section 16, including a sample collection section 13, are accumulated and integrally formed in a narrow detection space formed by covering a sensor support section 11 with a protective member. Therefore, miniaturization and weight saving are possible and also a small amount of a sample can be detected by a small amount of a reactant such as an antibody or an enzyme, and thus enabling a decrease in use amount of an expensive reactant can be reduced, and detection at low costs.

The ample delivery section 14 has a capillary structure, and one end of the capillary structure is close to a Peltier element. Thereby, it is possible to deliver a solution liquefied by the Peltier element through a capillary phenomenon of the capillary structure to a sample reaction section 15 without using a special structure.

As illustrated in Fig. 2(b), the sample reaction section 15 includes an antibody (enzyme-immobilized antibody) that specifically reacts with an analyte delivered through a sample delivery section 14. It is possible to specifically detect the analyte by an antigen-antibody reaction of the enzyme-immobilized antibody with the analyte.

As illustrated in Fig. 3(b), the detection section 16 includes an antibody 163 (substrate-immobilized antibody) that binds to an analytes reacted with a reactant (enzyme-immobilized antibody), and an electrode 162 that supports the antibody 163, and an electrode 162 has a porous structure. When the electrode 162 has a porous structure, it is possible to increase an immobilization area of the antibody 163, and thus enabling an increase in detection sensitivity.

### (Second Embodiment)

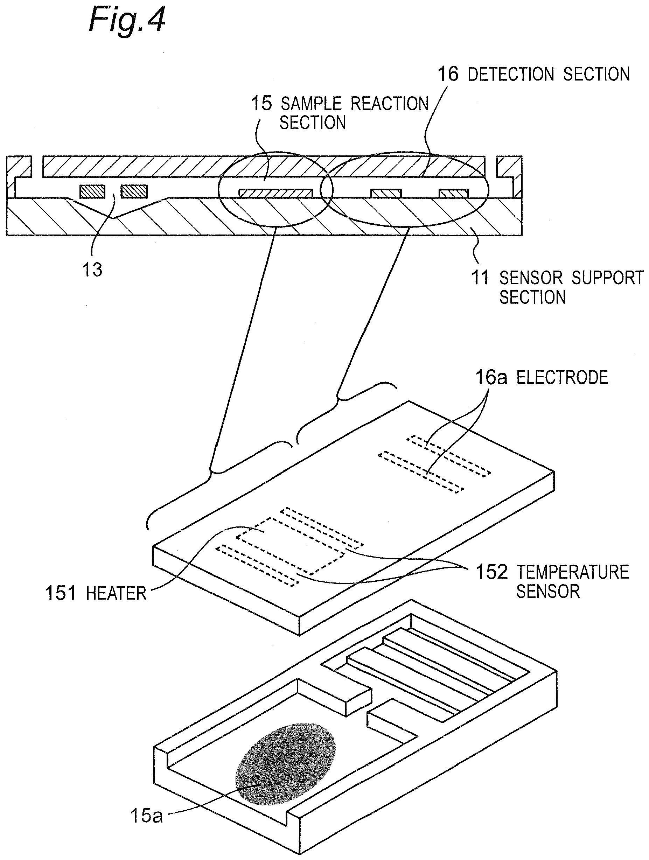

Specific configuration example of the sample reaction section 15 according to a second embodiment will be described with reference to Fig. 4(a) and Fig. 4(b). Since configurations excluding the sample reaction section 15 of a chemical sensor according to the second embodiment are the same as those in the first embodiment, illustration and description will be omitted.

Fig. 4(b) is an exploded perspective view of a sample reaction section 15 and a detection section 16. The sample reaction section 15 includes a heating section (for example, heater 151) in which a solution containing viruses delivered through a sample delivery section 14 is heated, and a temperature measurement section (for example, temperature sensor 152) in which the temperature of a solution containing viruses is measured. In the present embodiment, there is shown an example in which a heater 151 and a temperature sensor 152 are formed on a lower surface of an upper substrate that constitutes a protective member, and is provided in a detection space (sample reaction section 15) between the upper substrate and the sensor support section. Although the drawing is omitted, the sample reaction section 15 includes a control section in which a value of a current to be applied to the heater 151 is controlled based on the temperature of a solution measured by the temperature sensor 152. A plurality of heaters 151 and a plurality of temperature sensors 152 are respectively prepared, and they are respectively disposed in the sample reaction section 15 in a dispersed manner. On the lower surface of the upper substrate, an electrode 16a constituting a part of the detection section is formed.

In the sample reaction section 15, a primer, a DNA polymerase, deoxynucleotide triphosphate (dNTP) as a DNA synthesis material (substrate), and a buffer solution are disposed in a dry state.

In the control section, a polymerase chain reaction (PCR) method is carried out by controlling the heater 151. Herein, an example thereof is shown. DNAs in viruses are extracted by maintaining a solution containing viruses delivered to the sample reaction section 15 in the sample reaction section at high temperature, for example, about 90°C to 98°C. Next, the extracted DNAs are repeatedly exposed to high temperature of about 90 to 98°C and low temperature of about 50 to 65°C about 20 to 50 times. Thereby, it is possible to amplify DNAs as the analyte in the sample reaction section 15. The solution containing the amplified DNAs is conveyed to the detection section, where it is possible to confirm whether or not viruses are present in an initial solution by an oxidation-reduction reaction using an electrode. According to this PCR method, it is possible to detect the analyte with high sensitivity by amplifying a trace amount of the analyte.

While a method using an electrode has been shown as the detection method in the present embodiment, the same detection can be carried out even by an assay with fluorescence emitted by the amplified DNAs.

As illustrated in Fig. 4(b), the sample reaction section 15 includes a heating section (heater 151) in which an analyte delivered through a sample delivery section 14 is heated, and a temperature measurement section (temperature sensor 152) in which the temperature of the analyte is measured. Since a PCR method for amplifying DNAs of the analyte can be carried out by allowing a specific temperature cycle to occur using a heater 151, it is possible to detect the analyte with high sensitivity.

### (Third Embodiment)

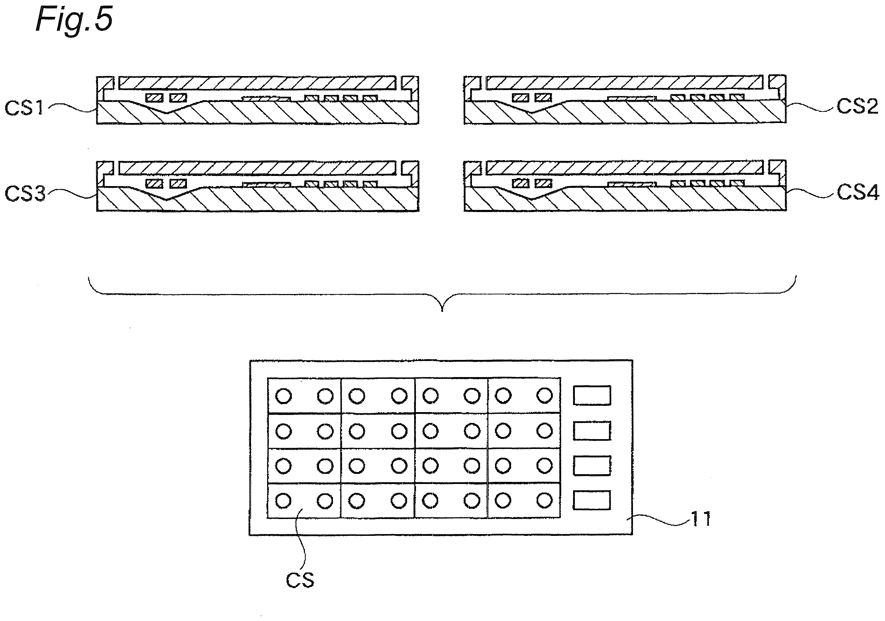

The entire configuration of a chemical sensor according to a third embodiment will be described with reference to Fig. 5. The chemical sensor according to the second embodiment is composed of a plurality of unit sensors CS1 to CS4 ... arranged into arrays. Each of unit sensors CS is composed of a gas injection hole 12, a sample collection section 13, a sample delivery section 14, a sample reaction section 15 and a detection section 16 illustrated in Fig. 1(a). A plurality of unit sensors CS are integrally formed on one sensor support section 11.

When a plurality of unit sensors CS are arranged in arrays on one sensor support section 11, it is possible to use only requisite chips (unit sensors CS) when needed, and thus enabling a decrease in number of replacements of the sensor, and suppression of single-use of the chip.

While the present invention has been described by way of three embodiments, it should not be understood that statements and drawings, that form a part of the disclosure, limit the present invention. Various alternate embodiments, Examples and operation technologies will become apparent to those skilled in the art from this disclosure.

Application examples of a chemical sensor according to embodiments of the present invention include home appliances added with a function of the above-mentioned chemical sensor, such as an air cleaner and an air conditioner; portable virus sensors in which the above-mentioned chemical sensor has been formed into a portable size by users; and portable devices added with a function of the above-mentioned chemical sensor, such as a cellular phone.

### [Reference Signs List]

- 11:: Sensor support section
- 12:: Gas injection hole
- 13:: Sample collection section
- 14:: Sample delivery section
- 15:: Sample reaction section
- 16:: Detection section
- 131:: Peltier element
- 151:: Heater (Heating section)
- 152:: Temperature sensor (Temperature measurement section)
- 162:: Electrode
- 163:: Antibody
- CS:: Unit sensor

## Claims

1. A chemical sensor comprising:
support section (11);
a gas injection hole (12) through which a gas containing an analyte is injected;
a sample collection section (13) in which the analyte contained in the gas injected through the gas injection hole (12) is collected;
a sample delivery section (14) through which the analyte collected in the sample collection section (13) is delivered;
a sample reaction section (15) including a reactant that causes a predetermined reaction with the analyte delivered through the sample delivery section; and
a detection section (16) in which the analyte reacted with the reactant in the sample reaction section (15) is detected;
wherein the sample collection section has a Peltier element that liquefies water vapor contained in the gas injected through the gas injection hole, and
wherein the sample reaction section (15) comprises a heating section (151) in which the analyte is heated, and a temperature measurement section (152) in which the temperature of the analyte is measured, and
wherein the heating section (151) and the temperature measurement section (152) are formed on a lower surface of an upper substrate that constitutes a protective member, the protective member being disposed over a main surface of the support section (11) so as to cover the sample collection section (13), the sample delivery section (14), the sample reaction section (15), and the detection section (16).

2. The chemical sensor according to claim 1, wherein the gas injection hole (12), the sample collection section (13), the sample delivery section (14), the sample reaction section (15) and the detection section (16) are integrally formed on the sensor support section (11).

3. The chemical sensor according to claim 1 or 2, wherein the sample delivery section (14) has a capillary structure, and one end of the capillary structure is close to the Peltier element (131).

4. The chemical sensor as in one of claims 1 to 3, wherein the sample reaction section (15) comprises at least any one of an antibody, an enzyme, a receptor and a protein, that specifically reacts with the analyte delivered through the sample delivery section (14) .

5. The chemical sensor as in one of claims 1 to 4, wherein the detection section (16) comprising an antibody (163) that binds to the analyte reacted with a reactant, and an electrode (162) that supports the antibody (163), the electrode (162) having a porous structure.

6. The chemical sensor as in one of claims 1 to 5, wherein a plurality of unit sensors (CS1 to CS4...) formed integrally on one sensor support section (11), each unit sensor (CS) comprising the gas injection hole (12), the sample collection section (13), the sample delivery section (14), the sample reaction section (15) and the detection section (16).

## Patentansprüche

1. Chemischer Sensor, aufweisend:
einen Stützabschnitt (11);
eine Gasinjektionsöffnung (12), durch die ein Gas injiziert wird, das einen Analyten enthält;
einen Probensammelabschnitt (13), in dem der Analyt, der in dem durch die Gasinjektionsöffnung (12) injizierten Gas enthalten ist, gesammelt wird;
einen Probenzufuhrabschnitt (14), durch den der in dem Probensammelabschnitt (13) gesammelte Analyt zugeführt wird;
einen Probenreaktionsabschnitt (15), der einen Reaktanten aufweist, der eine vorbestimmte Reaktion mit dem durch den Probenzufuhrabschnitt zugeführten Analyten hervorruft; und
ein Detektionsabschnitt (16), in dem der Analyt, der in dem Probenreaktionsabschnitt (15) mit dem Reaktanten reagiert hat, detektiert wird;
wobei der Probensammelabschnitt ein Peltier-Element aufweist, das Wasserdampf, der in dem durch die Gasinjektionsöffnung injizierten Gas enthalten ist, verflüssigt, und
wobei der Probenreaktionsabschnitt (15) einen Heizabschnitt (151), in dem der Analyt erhitzt wird, und einen Temperaturmessabschnitt (152), in dem die Temperatur des Analyten gemessen wird, aufweist, und
wobei der Heizabschnitt (151) und der Temperaturmessabschnitt (152) auf einer unteren Fläche eines oberen Substrats gebildet sind, das ein Schutzelement darstellt, wobei das Schutzelement so über einer Hauptfläche des Stützabschnitts (11) angeordnet ist, dass es den Probensammelabschnitt (13), den Probenzufuhrabschnitt (14), den Probenreaktionsabschnitt (15) und den Detektionsabschnitt (16) abdeckt.

2. Chemischer Sensor nach Anspruch 1, wobei die Gasinjektionsöffnung (12), der Probensammelabschnitt (13), der Probenzufuhrabschnitt (14), der Probenreaktionsabschnitt (15) und der Detektionsabschnitt (16) mit dem Sensorstützabschnitt (11) einstückig ausgebildet sind.

3. Chemischer Sensor nach Anspruch 1 oder 2, wobei der Probenzufuhrabschnitt (14) eine Kapillarstruktur aufweist und ein Ende der Kapillarstruktur sich nahe an dem Peltier-Element (131) befindet.

4. Chemischer Sensor nach einem der Ansprüche 1 bis 3, wobei der Probenreaktionsabschnitt (15) mindestens eines von einem Antikörper, einem Enzym, einem Rezeptor oder einem Protein aufweist, das spezifisch mit dem durch den Probenzufuhrabschnitt (14) zugeführten Analyten reagiert.

5. Chemischer Sensor nach einem der Ansprüche 1 bis 4, wobei der Detektionsabschnitt (16) einen Antikörper (163), der an den Analyten bindet, der mit einem Reaktanten zur Reaktion gebracht ist, und eine Elektrode (162), die den Antikörper (163) trägt, aufweist, wobei die Elektrode (162) eine poröse Struktur hat.

6. Chemischer Sensor nach einem der Ansprüche 1 bis 5, wobei mehrere Einheitssensoren (CS1 bis CS4...) einstückig auf einem Sensorstützabschnitt (11) ausgebildet ist, wobei jeder Einheitssensor (CS) die Gasinjektionsöffnung (12), den Probensammelabschnitt (13), den Probenzufuhrabschnitt (14), den Probenreaktionsabschnitt (15) und den Detektionsabschnitt (16) aufweist.

## Revendications

1. Capteur chimique comprenant :
une section de support (11) ;
un trou d'injection de gaz (12) à travers lequel un gaz contenant un analyte est injecté ;
une section de collecte d'échantillon (13) dans laquelle l'analyte contenu dans le gaz injecté à travers le trou d'injection de gaz (12) est collecté ;
une section de distribution d'échantillon (14) à travers laquelle l'analyte collecté dans la section de collecte d'échantillon (13) est distribué ;
une section de réaction d'échantillon (15) comprenant un réactif qui cause une réaction prédéterminée avec l'analyte distribué par l'intermédiaire de la section de distribution d'échantillon ; et
une section de détection (16) dans laquelle l'analyte ayant réagi avec le réactif dans la section de réaction d'échantillon (15) est détecté ;
dans lequel la section de collecte d'échantillon comporte un élément à effet Peltier qui liquéfie la vapeur d'eau contenue dans le gaz injecté à travers le trou d'injection de gaz, et
dans lequel la section de réaction d'échantillon (15) comprend une section de chauffage (151) dans laquelle l'analyte est chauffé, et une section de mesure de température (152) dans laquelle la température de l'analyte est mesurée, et
dans lequel la section de chauffage (151) et la section de mesure de température (152) sont formées sur une surface inférieure d'un substrat supérieur qui constitue un élément de protection, l'élément de protection étant disposé sur une surface principale de la section de support (11) de façon à recouvrir la section de collecte d'échantillon (13), la section de distribution d'échantillon (14), la section de réaction d'échantillon (15), et la section de détection (16).

2. Capteur chimique selon la revendication 1, dans lequel le trou d'injection de gaz (12), la section de collecte d'échantillon (13), la section de distribution d'échantillon (14), la section de réaction d'échantillon (15) et la section de détection (16) sont formées de façon intégrée sur la section de support de capteur (11).

3. Capteur chimique selon la revendication 1 ou 2, dans lequel la section de distribution d'échantillon (14) a une structure capillaire, et une extrémité de la structure capillaire est proche de l'élément à effet Peltier (131).

4. Capteur chimique selon l'une des revendications 1 à 3, dans lequel la section de réaction d'échantillon (15) comprend au moins l'un quelconque parmi un anticorps, une enzyme, un récepteur et une protéine, qui réagit spécifiquement avec l'analyte distribué par l'intermédiaire de la section de distribution d'échantillon (14).

5. Capteur chimique selon l'une des revendications 1 à 4, dans lequel la section de détection (16) comprend un anticorps (163) qui se lie à l'analyte ayant réagi avec un réactif, et une électrode (162) qui soutient l'anticorps (163), l'électrode (162) ayant une structure poreuse.

6. Capteur chimique selon l'une des revendications 1 à 5, dans lequel une pluralité de capteurs unitaires (CS1 à CS4...) sont formés de façon intégrée sur une section de support de capteur (11), chaque capteur unitaire (CS) comprenant le trou d'injection de gaz (12), la section de collecte d'échantillon (13), la section de distribution d'échantillon (14), la section de réaction d'échantillon (15) et la section de détection (16).
